# EUROPEAN PATENT SPECIFICATION

(11) **EP 1 327 001 B1**
(45) Date of publication and mention of the grant of the patent: **06.12.2006**
(21) Application number: 01955455.9
(22) Date of filing: 07.08.2001
(51) Int. Cl.: C12Q 1/68

(54) **METHOD OF AMPLIFYING NUCLEIC ACIDS PROVIDING MEANS FOR STRAND SEPARATION AND UNIVERSAL SEQUENCE TAGS**
VERFAHREN ZUR AMPLIFIKATION VON NUKLEINSÄUREN, DAS MITTEL ZUR STRANGTRENNUNG UND UNIVERSELLE SEQUENZMARKER BEREITSTELLT
PROCEDE D'AMPLIFICATION D'ACIDES NUCLEIQUES FOURNISSANT DES MOYENS DE SEPARATION DE BRINS ET DES MARQUEURS DE SEQUENCES UNIVERSELS

(30) Priority: 07.08.2000 GB 0019179
(43) Date of publication of application: 16.07.2003
(73) Proprietor: BioQuant Limited, Oxford Oxfordshire OX1 2AJ (GB)
(72) Inventor: POTTER, Collin, Gerald, c/o BioQuant Limited, Littlemore, Oxford OX4 4SS (GB)
(74) Representative: Woods, Geoffrey Corlett
(86) International application number: PCT/GB2001/003582
(87) International publication number: WO 2002/014534

(56) References cited:
- WO-A-92/17609
- WO-A-97/42345
- WO-A-98/14610
- WO-A-99/31272
- KEMP D J ET AL: "COLORIMETRIC DETECTION OF SPECIFIC DNA SEGMENTS AMPLIFIED BY POLYMERASE CHAIN REACTIONS" PROCEEDINGS OF THE NATIONAL ACADEMY OF SCIENCES OF USA, NATIONAL ACADEMY OF SCIENCE. WASHINGTON, US, vol. 86, no. 7, 1 April 1989 (1989-04-01), pages 2423-2427, XP000268576 ISSN: 0027-8424
- HEATH K.E ET AL.: "Universal primer quantitative fluorescent multiplex (UPQFM) PCR: a method to detect major and minor rearrangements of the low density lipoprotein receptor." JOURNAL OF MEDICAL GENETICS, vol. 37, no. 4, April 2000 (2000-04), pages 272-280, XP001055883 cited in the application

## Description

### Field of Invention

The invention relates to methods of amplifying DNA and methods of detecting, quantifying, purifying, isolating and manipulating the amplified DNA

### Background of Invention

Recent advances in genetics have made methods of genetic analysis important for many applications. These cover subjects such as the detection of the presence or absence of genetic traits by knowledge of a particular DNA sequence of an individual, the presence and abundance of particular organism such as a virus or other micro-organisms and the quantification of messenger RNA transcribed from the DNA. All these require robust and simple methods for their detection and quantification, preferably at low cost.

It is often difficult to work with material directly, especially in forensic work, for example, where only minuscule amounts of DNA may be available. For this and other purposes a biochemical amplification of nucleic acid is often carried out. One such system, the well-known polymerase chain reaction (PCR), is commonly used to amplify small numbers of target DNA molecules up to quantities that can be detected and characterised. The exact binding (or hybridisation) of short lengths of complementary single-stranded DNA (the primers) to either end of the target nucleic acid specifies the segment of DNA to be amplified by directing initiation of DNA synthesis. RNA may also be amplified by initially using reverse transcriptase in a process known as rtPCR to produce cDNA that can then be further amplified using PCR.

Present detection methods range from simple schemes that just detect the much larger quantities of DNA present after amplification to Southern blotting techniques whereby single strand DNAs are size-separated by gel electrophoresis and transferred to a membrane. A complementary strand, labelled in some way, is then used as a probe to hybridise specifically to the matching sequence. After washing away unattached molecules, the remaining probe is visualised. For critical work in forensic or clinical studies the simple methods are not rigorous enough. On the other hand the electrophoretic methods make for good validation but are not suitable for screening, being slow and hence expensive.

Between these extremes lie other techniques more suitable for high throughput where detection is carried out, either after separation of amplification products from the unused reagents, or by using a homogenous detection system without a separation step. Separation techniques generally require the use of a substrate such as plastic microtitration sample plates or beads to which the PCR product may be immobilised. In one technique the first PCR primer may have a chemical moiety such as biotin attached that can be used to bind the molecules to a plate or beads coated with a binding moiety such as streptavidin. Although it would be possible for the second primer to have an attached detectable label, this would then be present on the opposite strand from the biotinylated primer. It is therefore a principle that it is not normally possible to use both incorporated primers, each having useful properties such as a label or an attached binding moiety.

Detection may also be carried out through intrinsic labelling of the synthesised molecules during amplification or a labelled probe may be hybridised to a single stranded amplification immobilised product. In all these cases, sizing information is absent but the specificity of the single stranded DNA primer molecules, plus that of any hybridising probe, is adequate for most purposes.

Specified sequence arrays of DNA molecules may also be used to which the PCR product (or rtPCR-derived cDNA from mRNA) is allowed to hybridise. Visualisation of a labelled product or a probe hybridised thereto will show which sequence is present. Often such sequence arrays are proprietary.

Plate-based, semi-automated hybridisation techniques for viral quantification and polymorphism detection have been developed that use probes labelled with chelates containing rare earth elements such as Europium, Samarium or Terbium attached to short (usually12-15 base pair) oligonucleotides. These have the advantage of being non-toxic and non-radioactive. The protocols may be simplified to very few operations, despite this being a separation technique where the plates must be washed free of competing labelled oligonucleotides after hybridisation. The advantages are that all operations are carried out at room temperature, that two or even three probes with differing labels can be detected in the same well and, where two or three probes are used, one probe can act as an internal control for validating or standardising the reaction. The measurement by time-resolved fluorometry for single wells and for two probes takes only about 10 minutes per 96 samples. This technique benefits from very low background and good repeatability since the chelates are remarkably stable. Such specialised probes are however rather expensive although this cost is reasonable on an individual sample basis when required for high throughput. On the other hand, the cost makes it prohibitively expensive for detection of a multiplicity of micro-organisms or polymorphisms, each to be measured on a modest number of samples.

Radioactive, chemiluminescent or fluorescent labels may also be used in such assays for detection but these are often also expensive and may deteriorate with time and radioactive probes inevitably decrease in activity as the isotope decays. When ownership of many probes is contemplated it also has to be borne in mind that there are significant costs associated with initial optimisation of hybridisation conditions for each probe. The cost and logistics of keeping stocks up to date and in good order are also considerable.

The use of specialised single-strand primers has been described as state of the art where a long primer is used that overhangs the end of the target nucleic acid molecule after hybridisation. Nucleic acid synthesis completes this strand in the usual way but subsequent synthesis extends the strand to the end of the overhang thereby producing a known sequence at the end of the molecule. This process is very useful as special sites may be included such as restriction enzyme sites so that cutting will give molecules suitable for cloning or circularisation of the molecule. Sometimes the system is used to overcome difficulties with idiosyncratic sequences that are difficult to amplify and would otherwise produce significant amounts of incorrect amplification products. By using initial synthesis at low temperatures the specific aspect of primers binding is optimised while higher temperatures are used for later cycles that are suitable for the whole length of the primers. This produces a high stringency process overall so that the purity of products is improved (Weighardt F, Biamonti G and Riva S. "A simple procedure for enhancing PCR specificity". PCR Methods and Applications. 1993 Vol. 3, pp77-80).

Shuber A.P. shows a similar method in US Patent 5,882,856 of 16^{th} March 1999 (filed Jun 7^{th} 1995) entitled "Universal Primer Sequence for Multiplex DNA Amplification". Here multiple polymerase chain reactions are run simultaneously and the low temperatures for initial primer annealing define the required sequences for later amplification using the entire primer. This produces an even amplification and avoids problems of non-linearity that would otherwise produce a preponderance of some sequences to the detriment of the others that are equally important to the assay. Both these methods use gel electrophoresis for detection through size-separation of the molecules or their restriction enzyme digests. Alternatively, allele-specific probes are used to demonstrate the presence of the sequences of interest of techniques such as single-strand conformational polymorphism.

There is also an overhang detection method, marketed by Intergen Corp, but where the specification is confined to use of a probe in the form of a hairpin through self-complementary sequences. Hybridisation of the probe to an overhang allows synthesis that opens the hairpin by the extension process thereby separating a fluorescent emitter and a quencher, giving rise to fluorescence.

A primer incorporated into the end of a molecule can be useful for immobilisation of the molecule on a surface as described through attachment of a binding moiety such as biotin. The overhang can itself be used for attaching the molecules to a surface that bear single stranded DNA anchor sequences (Genosys Biotechnologies, Inc.) via a bridging oligonucleotide that will hybridise to both the anchor and the end-labelled amplified product. In this way many different types of samples may be assayed at the same time using identical anchor molecule-bearing wells in universal plates.

An overhang method has also been used for universal primers where the overhang portions extended during amplification are then subsequently amplified by a second pair of primers suited to the universal sequence of the overhangs ("A universal procedure for primer labelling of amplicons". Neilan BA, Wilton AN and Jacobs D. Nucleic Acids Research 1997 Vol. 25 pp2938-2939). A similar method was used with multiplex PCR samples to detect BRCA1 and BRCA2 deletions and insertions. Detection was by a procedure that used a third set of primers for a ligase reaction to produce a complete and labelled molecule only if the particular single nucleotide polymorphism was present in the amplified product. One ligase primer was labelled while the other had yet another overhang that could hybridise to oligonucleotides immobilised on an array. ("Universal DNA array detection of small insertions and deletions in BRCA1 and BRCA2". Favis R, Day JP, Gerry NP, Phelan C, Narod S and Barany F. in Nature biotechnology Vol.18, 561-564). A similar procedure was used but with detection by an automated sequencing electrophoresis apparatus. ("Universal primer quantitative fluorescent multiplex (UPQFM) PCR: a method to detect major and minor rearrangements of the low-density lipoprotein receptor gene". Heath KE, Day INM and Humphries SE. Journal of Molecular Genetics, Vol. 37, pp272-280).

WO97/42345 describes a method for detecting a target sequence using a first primer to introduce tag and detector sequences into an intermediate PCR product which is then further amplified using a primer complementary to the tag sequence. A probe comprising the detector sequence is used to detect the final PCR product.

WO99/31272 describes a method for detecting polymorphic restriction sites by introducing a tag sequence into a PCR product. The double-stranded PCR product is captured on a microarray via the tag sequence and restriction enzyme digestion is performed on the captured product.

### Summary of the invention

The invention described presents a solution to the need to use large numbers of different hybridisation probes in high-throughput screening but without sacrificing the specificity of the assays. The method is simple and convenient because the same probe or probes may be used for every polymorphism, micro-organism and messenger RNA quantification. The method of the invention uses universal primers, where one or both primers have overhanging DNA sequences not found in the sequence of interest. Upon DNA synthesis the complementary strand is extended to the end producing a new but known terminal sequence. Here it is the complementary sequence to the unrelated primer sequence that is used for detection by hybridisation to a universal probe having the same sequence as the primer overhang. Any competing primers and intermediate products may be removed by an alkali wash. The presence of label will therefore demonstrate that complementary DNA synthesis has occurred from the one primer to the end of the overhang region. This is good evidence that amplification has taken place as this has been directed by the specificity of the primers.

In the present invention the use of the overhanging sequences for direct detection and immobilisation gives a very considerable simplification for detection and quantification of nucleic acids. It also permits simple methods of isolation, purification and manipulation with the possibility of providing kits.

Accordingly the present invention provides:
- a method for detecting a target nucleic acid in a sample, which method comprises:
   (a) providing a primer pair comprising a first primer having a target specific sequence complementary to the sequence of the first strand of said target nucleic acid and an overhang sequence unrelated to said target nucleic acid sequence and a second primer having a target specific sequence complementary to the sequence of the second strand of said target nucleic acid and an attachment means;
   (b) contacting a nucleic acid sample with said first and second primers;
   (c) carrying out a polymerase chain reaction (PCR) under conditions suitable for the formation of a double-stranded product comprising the sequence of said target nucleic acid and having a first strand comprising said first primer and a second strand comprising said second primer and a sequence complementary to said overhang sequence of said first primer;
   (d) separating said first strand from said second strand using said attachment means; and then
   (e) detecting said second strand by binding a probe to the sequence in said second strand which sequence is complementary to the overhang sequence in said first primer or detecting said first strand by binding said overhang sequence in said first strand to an immobilised anchor sequence and detecting the immobilised first strand
      thereby detecting whether said target nucleic acid is present in said sample;
- a kit for detecting a target nucleic acid molecule, which kit comprises:
   (a) an oligonucleotide probe comprising the same sequence as an overhang sequence of a PCR primer,
   (b) an immobilisation surface;
   (c) a means for detecting said oligonucleotide probe;
   (d) a primer pair suitable for use in detecting a target nucleic acid, which primer pair comprises:
      (i) a first primer having a target specific sequence complementary to the sequence of the first strand of said target nucleic acid and an overhang sequence unrelated to said target nucleic acid sequence; and
      (ii) a second primer having a target specific sequence complementary to the sequence of the second strand of said target nucleic acid and an attachment means, wherein said attachment means is biotin; and
- a method as described above which further comprises selecting the overhang sequence of the primer by:
   (a) generating all possible sequences for a particular suitable length of overhang;
   (b) rejecting every sequence found within the sequence of interest;
   (c) optionally rejecting sequences having secondary structures;
   (d) selecting the remaining sequences for use in the generation of primers; and
- a kit for detecting a target nucleic acid molecule, which kit comprises:
   (a) an oligonucleotide probe comprising the same sequence as an overhang sequence of a first primer having a target specific sequence complementary to the sequence of the first strand of said target nucleic acid and an overhang sequence unrelated to said target nucleic acid sequence;
   (b) an immobilisation surface coated with streptavidin;
   (c) a primer pair suitable for use in detecting a target nucleic acid, which primer pair comprises:
      (i) a first primer having a target specific sequence complementary to the sequence of the first strand of said target nucleic acid and an overhang sequence unrelated to said target nucleic acid sequence; and
      (ii) a second primer having a target specific sequence complementary to the sequence of the second strand of said target nucleic acid and an attachment means, wherein said attachment means is biotin.

### Brief Description of the Drawings

### Key to the diagrams

N.B. I,II,II,IV describe the order of DNA strands synthesised.
1. +++++++++ part of first primer sequence-specific to end of product.
2. +++++++++ antisense sequence complementary to specific part of first primer.
3. ooooooooooo sense sequence of interest.
4. ooooooooooo antisense sequence of interest.
5. *********** second primer sequence specific to end of product
6. *********** sequence complementary to second specific primer sequence.
7. 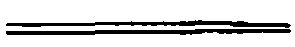 = overhang sequence of first primer with same sequence as probe.
8. 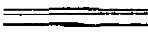 overhang sequence of first primer with complementary sequence to probe.
9. 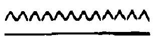 overhang sequence of second primer.
10. 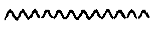 sequence complementary to overhang of second primer.
11.B - biotin.
12.Eu⁺⁺⁺ - Oligonucleotide label, may be lanthanide chelate (e.g. Eu, Sm, Tb or Dy) for time-resolved fluorometry, or radioactive, or chemiluminescent or fluorescent label.
13. +++++++++**X** part of first primer sequence specific to polymorphism at X.
14. ##########**Y** part of first primer sequence specific to polymorphism at Y.
15. Sm⁺⁺⁺ - Oligonucleotide as a second label.
16. 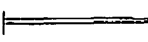 overhang sequence hybridising to anchor sequence.
17. ^{# # ## # # #} incorporated label - radioactive, fluorescent, colourimetric or chemiluminescent.

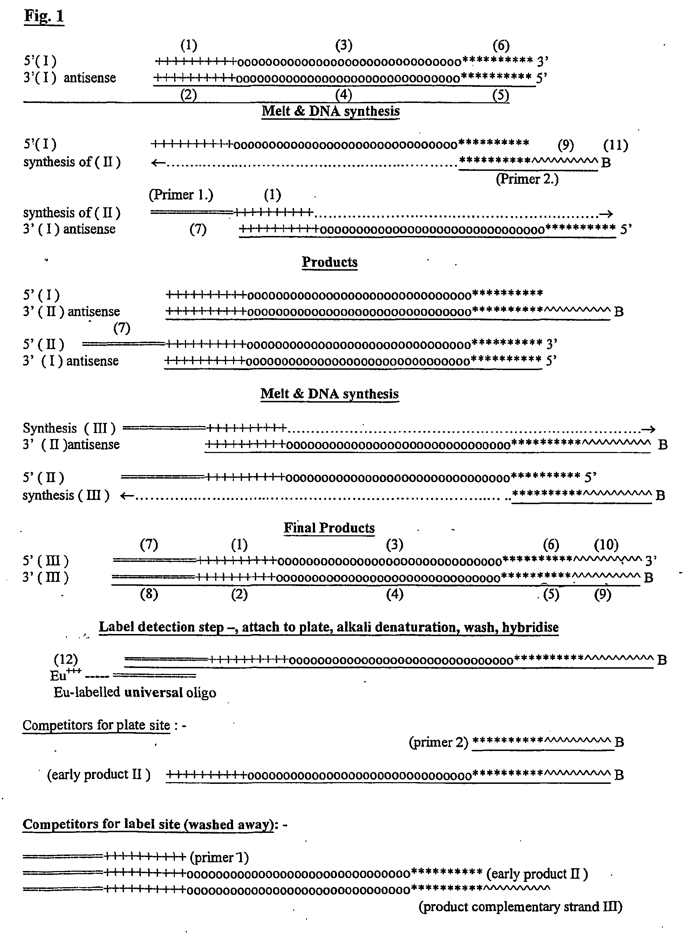

Figure 1 shows a PCR method for amplifying a target nucleic acid sequence (3) using overhanging primers, univeral probes and streptavidin plates. The first primer consists of a 5' overhang sequence (7) having the same sequence as that of the labelled probe (12) together with a 3' portion (1) specific to the sequence of interest. The second primer is biotinylated (B) and has a 3' overhang (9) with a 5' sequence specific portion (5). After PCR, products of the reaction (III) have biotin (B) at one end (originally attached to the end of the second primer), or other attachment means, and can be immobilised on streptavidin plates or beads. Quantification of the probe label bound to immobilised products will detect amplified product specified by the primers.

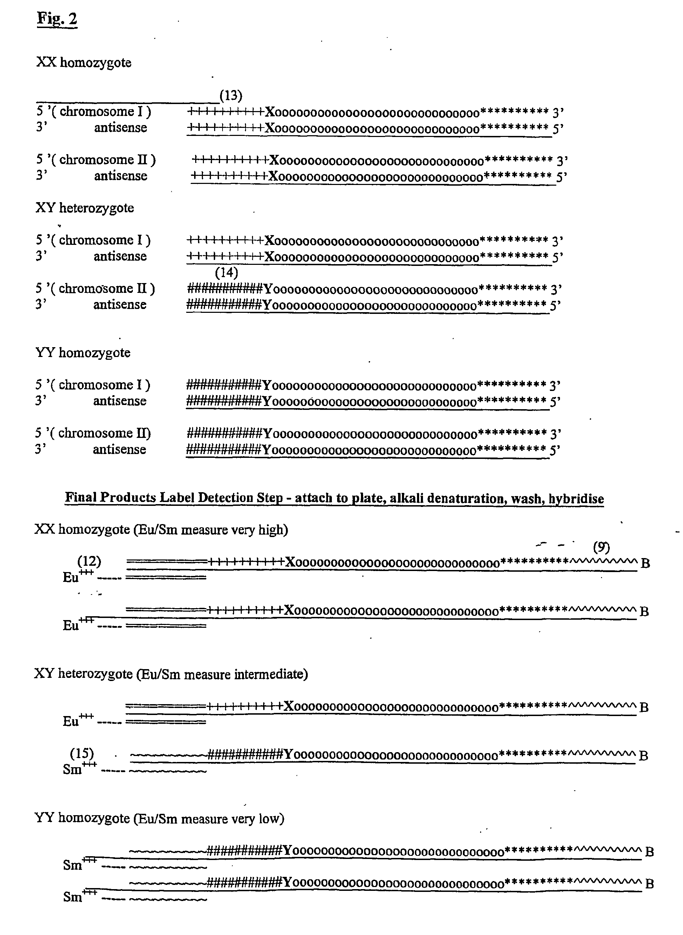

Figure 2 shows a variation of the method illustrated in Figure 1. The method illustrated in Figure 2 is for detecting single nucleotide polymorphisms using two differently labelled probes (for example, Eu and Sm labelled probes) for detecting PCR products. The amplitude refractory mutation system (ARMS) technique is used. The ratio of the measurements using the two probes will determine relative abundance of the polymorphic sequences and enable detection of heterozygosity as opposed to the homozygosity for either sequence.

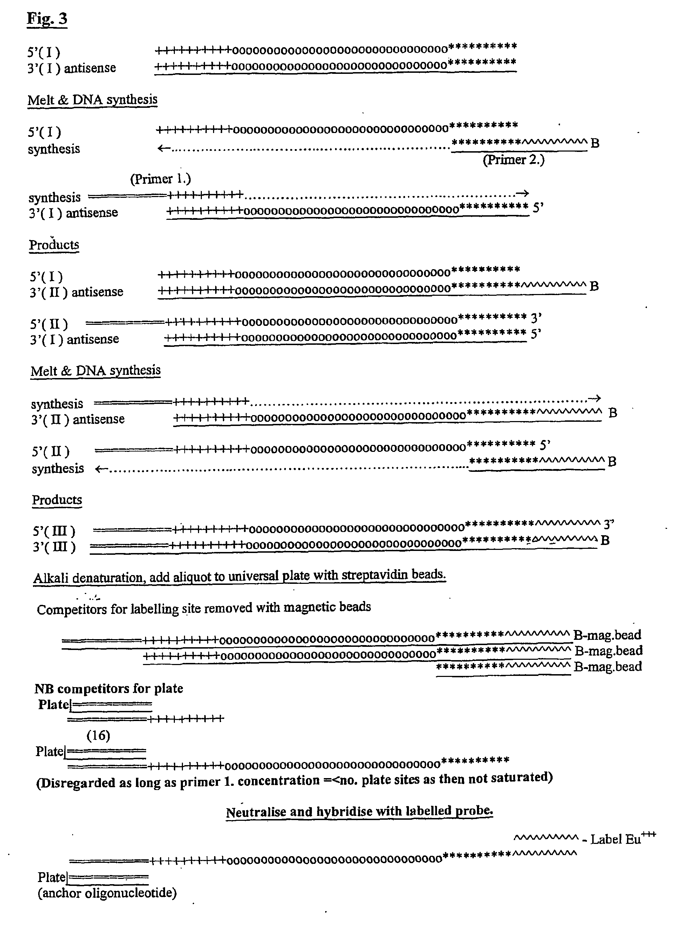

Figure 3 illustrates a method of the invention wherein overhang sequences are used on the both first and second primers. The overhang sequence on the second primer functions as an attachment means. This method may be cheaper to use than the methods shown in Figures 1 and 2 due to the fact that streptavidin-coated plates are expensive. The overhang on the second primer is made complementary to an anchor sequence (16) immobilised to a surface such as a plastic plate such that specific PCR products may be captured by hybridisation. The overhang on the first primer will then be available for detection by a universal probe at the distal end of the molecule as shown in Figure 1. This first primer may also be biotinylated (B), or may contain a similar attachment moiety; so that competing products after denaturation may be removed by attachment to streptavidin-coated beads, such as magnetic beads, or other such surface, followed by a washing step or use of magnetic fields. One advantage of this method is that the quantity of beads, or similar, is not critical for the assay excepting being adequate to remove the competing products.

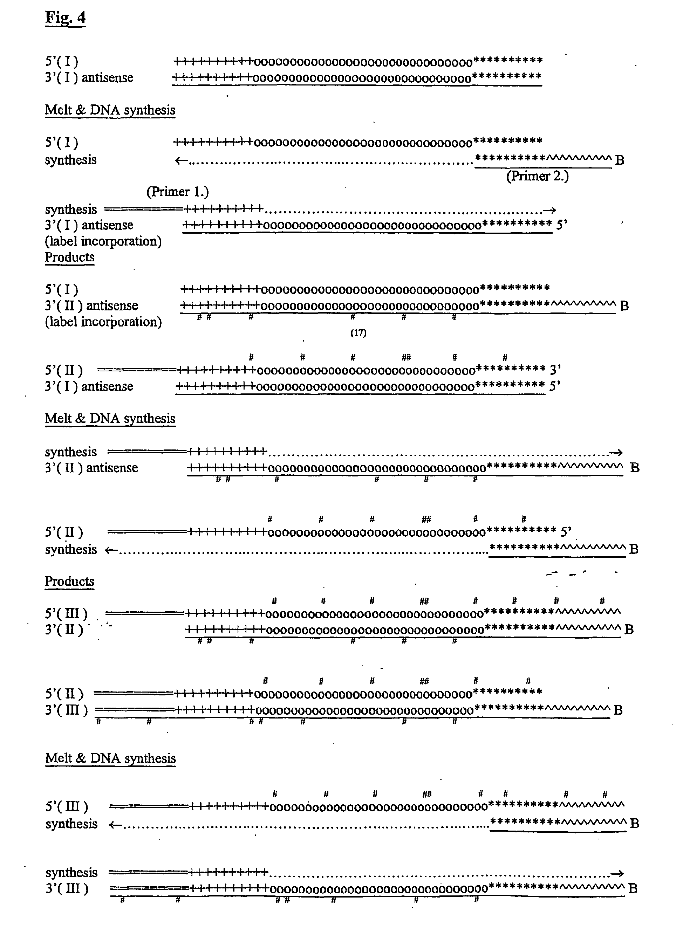

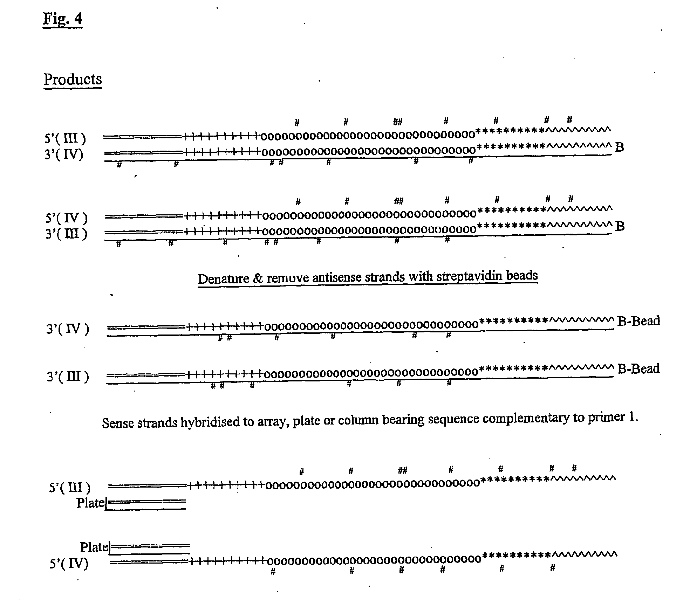

Figure 4 illustrates an embodiment of the invention where the DNA incorporates a label (17) during synthesis. Suitable labels include radioactive, colourimetric, chemiluminescent and fluorescent labels. The presence in the PCR product of an overhang with a sequence the same as that of an anchor sequence (16)will ensure that the labelled strand will hybridise to the immobilising surface. Competing products may be removed if biotin is attached to the other primer so that, after denaturation by heat or alkali, the strands without label may be removed by attachment to streptavidin-coated beads, such as magnetic beads, or other such surface using a washing or magnetic separation step. The remaining labelled strands may then be allowed to hybridise to anchor sequences and the label detected by standard methods.

### Detailed Description of the Invention

The present invention provides a method for detecting a target nucleic acid molecule in a sample. The method provided by the invention utilises universal detection methods which may be used to detect the presence of any target nucleic acid. The method provided by the invention utilises the well-known polymerase chain reaction (PCR) reaction to produce novel products. One strand of a double-stranded PCR product produced by a method of the invention has both a target-independent detection means at one end and a universal target-independent attachment means at the other. This enables a single strand of the PCR product to be immobilised and probed in a highly specific manner.

The present invention provides a method for detecting a target nucleic acid in a sample, which method comprises:
(a) providing a primer pair comprising a first primer having a target specific sequence complementary to the sequence of the first strand of said target nucleic acid and an overhang sequence unrelated to said target nucleic acid sequence and a second primer having a target specific sequence complementary to the sequence of the second strand of said target nucleic acid and an attachment means;
(b) contacting a nucleic acid sample with said first and second primers;
(c) carrying out a polymerase chain reaction (PCR) under conditions suitable for the formation of a product having said attachment means and a sequence comprising the sequence of said target nucleic acid and the sequence of said overhang;
(f) separating first strand of said product comprising said first primer from second strand of said product comprising said second primer using said attachment means; and
(g) detecting said first strand or said second strand
thereby detecting whether said target nucleic acid is present in said sample.

### Primers

The invention provides a primer pair suitable for use in a method of detecting a target nucleic acid of the invention, which primer pair comprises:
(i) a first primer having a target specific sequence complementary to the sequence of the first strand of said target nucleic acid and an overhang sequence unrelated to said target nucleic acid sequence; and
(ii) a second primer having a target specific sequence complementary to the sequence of the second strand of said target nucleic acid and an attachment means.

The first and second primers are designed to flank the target nucleic acid sequence of interest.

The first primer sequence typically comprises a total of from 15 to 50 bases, preferably from 25 to 40 bases. The overhang sequence is typically from 8 to 20 bases, preferably from 10 to 16 bases.

The overhang sequence is generally chosen so that it is a sequence that does not exist in the sample being tested. The overhang sequence may be chosen so that it is a sequence that is not present in the genome of the organism or organisms of interest. Preferably, the overhang sequence is chosen so that it is a sequence which does not appear in the genome of any organism. The overhang sequence may be selected using suitable algorithms or may be selected by experimental means. Suitable assays for determining whether a defined sequence is present in a target will be apparent to a person skilled in the art. A method for the selecting suitable sequences that are not present in genomes is described herein.

The overhang sequence is also preferably chosen so that neither the overhang sequence nor the sequence complementary thereto forms a hairpin loop or other secondary structure. The overhang sequence may comprise a restriction enzyme site.

The second primer sequence typically comprises a total of from 15 to 50 bases, preferably from 25 to 40 bases with the overhang sequence typically comprising from 0 to 20 bases, preferably from 10 to 16 bases.

The second primer comprises an attachment means which may function to anchor one strand of the PCR product to a surface. The anchored strand may then be detected using a probe specific for the overhang sequence at the other end of the PCR product. Alternatively, the strand not anchored to the surface may be detected.

Any suitable attachment means may be used. The attachment means on the second primer may comprise one member of a specific binding pair, for example a biotin moiety which will bind to a surface coated with streptavidin. The attachment means may comprise an overhang sequence which is complementary to an anchor sequence. Where the attachment means is an overhang sequence, the strand of the PCR product comprising the second primer is preferably captured by binding to a surface on which oligonucleotides comprising the anchor sequence are immobilised.

The second primer may comprise an overhang region. The overhang region may be used as an attachment means. Alternatively the overhang region can be present in addition to a further attachment means such as a biotin moiety.

Overhang sequences on both the first or the first and second primers are typically chosen such that the melting points of the first and second primers are reasonably matched after synthesis of any overhangs present, i.e. so that the melting point of the whole first primer is approximately the same as the melting point of the whole second primer.

The first primer may further comprise an attachment means. The attachment means on the first primer may function to aid removal of the primer and single stranded products of the PCR products comprising the first primer (the first strands) prior to detection of the single stranded products comprising the second primer (the second strands). Alternatively, the attachment means on the second primer may first be used to remove the second strands. The first strands may then be attached via the attachment means on the first primer to a second surface prior to detection of the first strands.

Any suitable attachment means may be used. For example, the attachment means on the first primer may comprise one member of a specific binding pair such as a biotin moiety which will bind to streptavidin. The first primer may then be removed by the binding of the binding moiety to a suitable binding partner or anchor sequence. For example, if the first primer comprises biotin, it may be removed by binding to a streptavidin-coated surface such as streptavidin-coated beads.

The attachment means may comprise an overhang sequence which is complementary to an anchor sequence. The attachment means may comprise all or part of the overhang sequence of the first primer. It is preferred that the attachment means comprises an overhang sequence when the second strands are removed and detection is of the first strands.

One half of a specific binding pair, such as streptavidin or an anchor sequence may be immobilised on the surface of beads. The beads to which either the first or second strand has been attached may be removed by centrifugation or by magnetic means.

The first primer and said second primer may comprise the same attachment means or different attachment means. Where the first and second primers comprise the same attachment means, the double stranded PCR product may be denatured and the single strands may be separated by binding to the same surface using the attachment means of each of the first and second primers. Where the first and second primers comprise the same attachment means it is preferred that the attachment means is a member of a binding pair. The present invention also provides a set of primers comprising two or more said primer pairs for the simultaneous detection of two or more target nucleic acids. The overhang sequences of each of the primers are typically selected so that as the melting point (Tm) of the double strand is similar for each of the primers used and is within a range suitable for the amplification process. The selection of suitable conditions is described in more detail below.

Each first primer in said set may comprise a different overhang sequence and a different target specific sequence and each second primer in said set may comprise a different target specific sequence but the same attachment means. Thus, in step (e) of a method of the invention, each of the products produced using each of the primer pairs may be detected using a different labelled probe, each different probe having a different sequence and being labelled with a different label.

Alternatively, each first primer in said set may have the same overhang sequence but a different target specific sequence and each second primer in said set may comprise a different target specific sequence and a different attachment means. In this embodiment, the attachment means are preferably overhang sequences. Thus each primer pair will produce a product having a different but known overhang at one end and the same known overhang at the other end. The overhang which differs between the products of different primer sets may be used to attach the primers to different surfaces, or different parts of the same surface, using anchor sequences specific for each of the overhang sequences. The same probe may then be used to detect the products of all primer pairs and the products distinguished according to their positions of attachment.

### Samples

Any suitable nucleic acid sample may be used in a method of the invention. The sample used will depend on the target nucleic acid(s). The nucleic acid sample may comprise, for example RNA or DNA such as genomic DNA or cDNA.

For example, if method of the invention a method of genetic screening, for example the method may be aimed at determining the presence of a single nucleotide polymorphism (SNP) in a sample from an individual, the sample is preferably a sample of genomic DNA from the individual but may also be a cDNA or RNA sample derived from cells or tissues that express the gene comprising the sequence of interest.

The method of the invention may be used to detect, quantify or identify a particular microorganism. In this embodiment, the sample is preferably a genomic DNA sample, or a RNA sample. The RNA sample may, for example, be a sample of ribosomal rRNA from a microorganism.

For nucleic acid quantification, for example for gene counting or to determine micro-organism abundance, it is of great benefit if a different target nucleic acid is present in the same sample during amplification to act as a separately detectable internal control.

### Amplification Reaction

The initial reaction conditions such as temperature are preferably selected so that they are suitable for annealing of the sequence-specific portions of the primers to the target sequence in a sequence specific manner. Such conditions may readily be determined by a person skilled in the art. The initial reaction conditions may be required for at least the first 2 cycles, such as the first 3 to 20, 4 to 18 or 5 to 15 cycles, preferably 7 to 12 cycles, in order to accumulate statistically adequate numbers of molecules even if starting from one molecule in the original DNA sample. After enough extended products incorporating overhangs are accumulated, for subsequent cycles of amplification the annealing temperatures may be increased to suitable levels for the complete primers, including overhangs since these will have a higher melting point than the short sequence-specific portions engaged initially. The person skilled in the art will readily be able to determine suitable reaction conditions using well-known techniques.

Where simultaneous detection of two or more nucleic acids is carried out using two or more primer pairs the PCR reactions may be carried out in the same reaction vessel. It is preferred that the PCR reactions using the different primer pairs are carried out in the same reaction vessel. If the method is being used to determine the relative abundance of two or more target nucleic acids from a sample it is particularly preferred that the primer pairs are all present in the same reaction. Performing a method for the detection of more than one sequence in the-same vessel is preferred because it gives reduced probability of errors. This is because the amplification process is near exponential and can result in very different quantities of product produced from very small differences in the starting materials. The reaction is also very sensitive to small differences in conditions of the reaction such as the temperatures and chemical constitution of the reactants and these may vary for different wells during the amplification process.

In one embodiment, a label is added to the PCR reaction, for example radioabelled or fluorescently labelled nucleotides, such that the label is incorporated into the PCR products. Other suitable labels include colourimetric and chemiluminescent labels.

The present invention also provides a PCR product having a sequence comprising:
(a) a sequence identical to the sequence a nucleic acid amplified by the PCR;
(b) a sequence complementary to the overhang sequence of a first primer used in the PCR, which sequence is complementary to a probe; and
(c) a second primer comprising an attachment means used in the PCR.

### Separation

The two strands of the double-stranded PCR product produced in a method of the invention are typically separated using the attachment means present on the second primer or the attachment means present on the first and second primers. Either the first strand, i.e. the strand comprising the first primer, or the second strand, i.e. the strand comprising the second primer, may be used for further analysis.

The strand which is not being used for detection of the PCR product, intermediate products and primers that are the same as those incorporated into the PCR product not being used for detection are typically removed prior to detection to prevent them competing with the probe(s) used for detection.

The PCR products are preferably denatured, for example using 100-400mM sodium hydroxide or by heating to melt the strands. This may be carried out before or after the binding of one or both of the strands to a surface via attachment means incorporated into the primers used to amplify the strands. After said attachment has occurred, unbound nucleic acid may be removed by washing. The strand of interest may remain attached to the surface and ready for detection, for example by hybridisation with a probe. Alternatively, the strand of interest may be the strand which is not bound to the surface. This strand may then be attached to a second surface for analysis.

Both primers may comprise the same attachment means, for example both may be biotinylated, so that all the synthesised molecules and remaining primers will become bound to the surface and will not be in a position to compete with the probe. In this instance, a denaturing step is performed prior to attachment to the surface. This is conditional on the number of binding sites on the bead or well or other surface being equal to or greater than the number of molecules with attached binding moieties in the reaction aliquot added. Removal of competing oligonucleotides typically reduces the risk of non-linearity as would occur if any smaller molecules with higher mobility than the product of interest were to preferentially occupy the available binding sites.

The first primer may comprise a different attachment means to the second primer so that, following denaturation the two strands of the PCR product may be separated by selective removal of the undetcted strand. For example, if the strand that is not for use in the detection step is biotinylated, competing products may be removed after denaturation by attachment to streptavidin-coated beads, or other such surface followed by a washing step. The beads may be magnetic, in which case, following incubation of the beads with the sample of amplified nucleic acids, the beads may be removed using magnetic fields. The attachment means on the primer which will be incorporated into the undetected strand may also be magnetic such that the undetected products may be removed by attaching to a magnetic surface. One advantage of this method is that the quantity of beads is not critical for the assay excepting being adequate to remove the competing products.

One strand of the PCR product will typically comprise:
(a) a sequence identical to the sequence of the target nucleic acid;
(b) a sequence complementary to the overhang sequence of the first PCR primer; and
(c) a second PCR primer and attachment means.

Generally separation will be carried out such that this strand may be detected using a universal probe. The second PCR primer comprises an attachment means. Accordingly, one strand of the PCR product will comprise the same attachment means at its end. This strand, referred to herein as the second strand, may be immobilised on a surface via the attachment means.

Any suitable surface may be used, for example, a plastic multiwell plate, such as a microtitre plate, or beads, such as magnetic beads, may be used. The surface will generally be coated with a binding partner of the attachment means. For example, where the attachment means is a biotin moiety, the PCR product may be immobilised on a streptavidin-coated surface. Where the method of the invention is being used for the detection of two or more target nucleic acids, the PCR products will be generated using different primer pairs and may thus include different attachment means to separate the different products. Where biotin is the attachment means on one product another binding means may be present on the other product(s) and they may be bound to wells coated with suitable specific binding substances.

Where the second primer comprises an overhang sequence, the overhang sequence will be incorporated into one strand of the PCR product. The overhang sequence may be used to attach the product to a surface by binding to an anchor sequence which is complementary to the overhang sequence. The product may thus be attached to the surface (captured) by hybridisation. The product may be bound to an oligonucleotide comprising the overhang sequence prior to immobilisation of the oligonucleotide. It is preferred that the product is bound to oligonucleotide that is already immobilised. A number of different complementing anchor molecules and overhang sequences may be utilised to enable the rapid and simultaneous detection of multiple target nucleic acid sequences. Each second strand produced using each of the primer pairs is attached to a different surface. Each different surface may have a different anchor oligonucleotide immobilised thereon. The different surfaces may be separate wells in a multiwell plate or separate spots on an array.

Different but known anchor sequences may be immobilised in separate wells of a plate or placed in an array forming a "zip-code". Each sequence on the plate or array would therefore be known. In multiplex PCR, where a number of sequences are amplified at the same time, the primers would be synthesised to suit both the target DNA and the immobilised anchor molecules on the plate. Each amplified product will typically hybridise specifically to a particular well (or position in an array) and, therefore, all products may be detected using the same probe or probes throughout and using exactly the same hybridisation protocol. This is in contrast to usual array techniques where the sequence used must be picked to allow for the uniform hybridisation conditions required or allowance made for different degrees of hybridisation through standardisation.

Use of the overhang sequence on the second primer as an attachment means is also advantageous over the use of a biotin-steptavidin system in that it is cheaper to use due to the fact that streptavidin-coated plates are expensive.

Overhang regions for use as attachment means and oligonucleotides comprising anchor sequences are typically selected to have similar melting temperatures as well as being unrelated to the sequence of interest. Preferably the anchor oligonucleotides have similar melting temperatures to the probes used so that simultaneous incubation is possible. Such plates or arrays are universal and may be made in bulk very cheaply. In the laboratory, stocks of plates and the few universal probes needed may be the same for many different target nucleic acids. Only the specific primers (which store well, are quickly obtainable and cheap) are specific to the choice of target nucleic acid. Another major advantage of using the same probe is that the sequence and hybridisation conditions may be optimised and the same protocol may be used for many different assays. Kits may be produced with high consistency of results guaranteed. Once optimum primer sequences are worked out for each system, reliable genetic screening and micro-organism quantification may be carried out with only modest levels of skill or by automation.

One strand of the PCR product will typically comprise:
(a) a sequence identical to the sequence of the target nucleic acid;
(b) a sequence complementary to the overhang sequence of the first PCR primer; and
(d) a second PCR primer and attachment means.

The separation step may be carried out so that this strand is removed via the attachment means and the complementary strand having a sequence comprising:
(a) a sequence identical to the sequence of the target nucleic-acid;
(b) the first PCR primer; and
(e) a sequence complementary to the second PCR primer
is used in the detection step. In this embodiment the overhang sequence of the first primer in the single stranded product is used to both immobilise the product and as a target for a probe. The probe used is an oligonucleotide comprising a sequence which is complementary to the overhang sequence of the first primer. The probe is immobilised to a surface and so functions as an anchor sequence in addition to a probe. The position of a product on a surface will depend on the position of the anchor sequence complementary to the first primer used to amplify the product and hence multiple targets may be detected simultaneously. Detection in this embodiment is preferably by detection of a label incorporated into the product during the amplification reaction.

### Detection

Immobilisation of the second strand of the PCR product on a surface via the attachment means of the second primer typically serves to present the sequence at the distal end of the molecule which is complementary to the overhang on the first primer for detection by a universal probe. Alternatively, immobilisation of the second strand of the PCR product on a surface via the attachment means of the second primer may serve to remove the second strand, thus enabling the first strand to be further analysed. The first strand is typically analysed further by binding to immobilised probes functioning as anchor sequences.

Typically the second strand is detected. Detection is generally carried out using a probe comprising a sequence identical to the sequence of the overhang in said first primer.

The probe may comprise a detectable label. Any suitable detectable label may be used, for example a fluorescent, chemiluminescent, colourimetric or radioactive label. Preferably the label is a chelate containing a rare earth element such as Europium, Samarium or Terbium. Detection of the label may be by radioactive, fluorescent, chemiluminescent, colourimetric or time-resolved fluorescent means.

The detecting step typically comprises binding said probe to said second - strand of product and monitoring said binding.

In an embodiment where the attachment means on the second primer is an overhang sequence and multiple target nucleic acids are amplified in the same assay, each said second strand produced using each of the primer pairs may be detected using the same probe but will be bound to different anchor oligonucleotides at defined positions on a surface and so may be distinguished on the basis of the position on the surface to which they are attached.

Alternatively, where it is desired to detect the presence of two or more target nucleic acid molecules in a sample, co-amplification of two or more target sequences may be bound to the same surface and detected using different probes. Hence, the multiple target sequences may be detected in the same sample well. This may be used for example in an assay for determining the relative abundance of two or more target nucleic acids. In this embodiment, one target sequence is produced using a first primer with a first overhang sequence and a second target sequence is produced using a first primer with a second overhang sequence. A probe comprising a sequence identical to the first overhang sequence and a first label is used to detect the first target sequence and a probe comprising a sequence identical to the second overhang sequence and a second label is used to detect the second target sequence. The ratio of measurement of the labels will give the relative abundance of the first and second target sequences and, if compared to that of known standards processed in parallel in the same sample plate, will allow quantification of the nucleic acid(s) present.

Two primer pairs may be used to amplify two variants of a single nucleotide polymorphism (SNP), and the proportions of product produced using each of the primer pairs is monitored to determine whether said individual is homozygous or heterozygous for said SNP. In a preferred embodiment, PCR amplification of a target sequence is prevented where there is one or more mismatch between the target nucleic acid sequence and the target specific sequence of a primer. Typically, a mismatch at the internal end of a primer will inhibit synthesis and hence prevent amplification whereas perfectly matched primers will result in detectable product. For example, the amplitude refractory mutation system (ARMS) technique or the technique described in European patent No. 0 332 435 may be used. In the ARMS technique, primers which end 3' at a mutation site and also have a penultimate base unmatched will not initiate DNA synthesis, but a single mismatched site will initiate synthesis. Methods for selecting suitable mismatch primers will be readily apparent to a person skilled in the art.

Where the first strand of the PCR product is detected following removal of the second strand, the first strand is typically bound via the overhang sequence on the first primer to an anchor sequence immobilised on a surface. Binding is then detected by detecting a label that has been incorporated into the PCR product during PCR amplification. The label may be a radioactive, colourimetric, chemiluminescent or fluorescent label and may be detected by any suitable means known in the art. For simultaneous detection of multiple target sequences, the overhang sequence on each first primer in a set of primers is a different sequence. For each target sequence the first strand of the product will be complementary to a different anchor sequence. The anchor sequences may be arranged in a known pattern on a surface. The products for all target sequences will contain the same label and so can be detected in the same manner. The pattern of labelling will enable the target sequences present to be distinguished.

Where the overhang sequence comprises a restriction enzyme site, the probe may comprise a fluorescer and a quenching agent, which quenching agent reduces the light output from the fluorescer upon excitation. The fluorescer and quenching agent are typically positioned such that they are physically separated by the action of a restriction enzyme, i.e. they are located on opposite sides of the restriction enzyme cleavage site. In this embodiment, incubation of the PCR products and probe(s) with a suitable restriction enzyme will result in fluorescence only if the target nucleic acid was present in the sample. If the probe and restriction enzyme are added at the end of the PCR amplification, no denaturation step will be required and the resulting fluorescence will be proportional to the quantity of the desired product.

Where multiple target sequences are detected simultaneously, the overhang sequences in each of the first primers in a primer set may comprise a different restriction enzyme sequence. The target sequences present in a sample may then be distinguished on the basis of which restriction enzyme(s) produce a fluorescent product.

### Kits

The present invention provides kits for carrying out the method of the invention. A kit according to the invention typically comprises:
(a) an oligonucleotide probe comprising the same sequence as an overhang sequence of a PCR primer;
(b) an immobilisation surface;
(c) a means for detecting said oligonucleotide probe; and
(d) optionally a primer pair suitable for use in detecting a target nucleic acid, which primer pair comprises:
   (i) a first primer having a target specific sequence complementary to the sequence of the first strand of said target nucleic acid and an overhang sequence unrelated to said target nucleic acid sequence; and
   (ii) a second primer having a target specific sequence complementary to the sequence of the second strand of said target nucleic acid and an attachment means.

### Generation of nucleic acid sequences not present in genomes

In order to carry out the invention method, DNA sequences for both the universal overhang and specific inner portions of the primers will need to be devised. The genetic sequence is composed of the four bases cytosine, thymine, guanine and adenine. The length of a sequence varies according to the organism, for example, the human genome contains about 3E⁹ bases.

If the DNA sequence devised were random, then the total number of possible sequences for a probe of length x bases will be of 4^{x}. For example, for a probe of length 10 (10mer), there will be 4¹⁰=~1E⁶ possible different sequences. In the human genome 3E⁹/1E⁶ = 3000 10mer sites will be present by chance alone. Such a sequence would be useless for probing the whole human genome. For a random sequence probe 20 bases long however there are 4²⁰ = ~1E¹² possible and hence only 3E⁹/1E⁹¹² = .003 of a site expected by chance alone in the human genome. For a single probe this would be acceptable but now, sequences are often immobilised as an array of many thousand separate spots and mixtures of nucleic acids applied and spurious hybridisation could give rise to false positive results.

It is a recognised method whereby a series of sequences are immobilised to the wells of a plate or form an array, and this is sometimes known as a zip code technique. In the present invention, the sequences need to be known to be absent from the genome or genomes of interest and will then constitute a universal array or plate without danger of spurious hybridisation.

In such a case, multiplex PCR could add a suitable zip code using one end of the PCR products and general incorporation of label or the use of a labelled primer capable of hybridising to the other end may be used to detect the target nucleic acids present. A mixture, which may include consensus sequence(s) with different label(s) as controls, may be applied to the plate or array, washed and hybridised to give label at each position selected by the zip code and by the sequence specified

The present invention requires a selection method for each oligonucleotide to be considered for the overhang technique. As a first step all possible sequences are generated for a particular suitable length of oligonucleotide. Every sequence found within the sequence(s) of interest are then rejected. The sequence of interest may include the whole genome concerned. A list of oligonucleotides not present in the PCR product or the genome is thereby generated and use of these is necessary for the present invention. Many of these can be rejected through having secondary structures such when the oligonucleotide hybridises to itself producing a hairpin and such selection is well known to those skilled in the art. Some of these can also be rejected if the 3' ends of one oligonucleotide are found to complementary to the 5' end of the other oligonucleotide, as this makes the combination prone to undesirable primer-dimer formation.

The initial amplification used in this invention uses the inner portions of the primers that are specific to the sequence to be amplified. After full-length amplification products are formed the complete primers are thereon involved. The melting point of the inner portions of both primers will therefore need to be matched to set the annealing temperature for at least the first two amplification cycles; usually a few degrees below the melting point. The melting points of the entire primers will also need to be matched for subsequent amplification with correspondingly higher annealing temperatures. This has the effect of preventing further amplification of target sequences using the inner portions of the primers and therefore reduces the chance of spurious products. Any sequence has a characteristic melting point that can be calculated by well-known algorithms according to the proportion of different bases present. Oligonucleotide pairs for use in the overhang PCR detection system can therefore be designed to have similar melting points for both the inner portions and the entire primer length. These can be formulated by conventional methods by those skilled in the art.

Considering the present invention, if say an inner primer is 12 bases long then 4¹² in 3.1x10⁹ bases will give about 185 sites in the whole genome. This will mean that a site will on average be every 1.7 million bases which makes it unlikely that amplification could extend in the first two cycles from a chosen site to an unwanted complementary primer site and so produce spurious amplification products. If the entire primer is say 24 bases in length then the further amplification is most unlikely to produce any spurious products per se. It might be thought that the length of the primers, both inner and universal portions could be increased until there is very little chance that unsuitable annealing could take place. The whole length of the primer would then have a melting point that that could exceed the optimum temperature for the DNA polymerase and even approach the temperature required for melting the target DNA. These limits are well known to those skilled in the art.

This invention therefore provides a method for the generation of oligonucleotides not present in the sequence of interest, whether it is a PCR product or a genome but that are suitable for use as overhang-primers in the universal probe system.

The following Examples illustrate the invention.

### Example 1: Assay conditions - plates background linearity and capacity

Several parts of the invention require immobilisation of DNA products to streptavidin-coated surfaces, for example, plastic plates or beads that may also be magnetic. Three different plates were investigated for their capacity to bind using a 12-long (12mer) random nucleotide test probe with biotin at the 3' end and a labelled 5' end: The label was Europium atoms trapped in chelates covalently linked to a tail of modified cytidines on the 5' end of the probe (5' Eu (20 modified cytosines) TAATAAGACATCC- Biotin 3'(SEQ ID NO: 1)). Such atoms exhibit a long fluorescence when treated with an enhancement solution containing another chelate and can be measured by suitable plate readers by a time-resolved fluorometry technique that is well known to those skilled in the art. In the examples described below, any suitable label could be used, as the invention does not depend on the particular type of label employed.

Three commercial streptavidin plates were tested by shaking probe in 100µl commercial (Wallac Oy, Finland) TRIS buffer, diluted 1:1 with deionised water and with the addition of 2M NaCl, at pH 8.4 for 40 mins at room temperature (RT), washed 6x with Wallac wash solution. Enhancement solution was added and the plate shaken for 30 min. At high concentrations the amount of time-resolved fluorescence would excede the capability of the instrument (>30million counts per second) so a fixed concentration of probe (10fmol) was used but diluted with plain unlabelled streptavidin. Although the molecular weights were very different and substitution should not be strictly correct, the shaking ensured distribution of the molecules and even binding. The result was that the fluorescence was linear up to the capacity of the particular plate as shown in Table 1. Backgrounds were also measured and varied between the different plates. Plate A was low backgound but unstable, comparing immediate counting with counting 30 mins later. Plate B had a low background and was quite stable and Plate C was high background but very stable. Plate A had limited capacity (~1pmol) while Plates B and C could bind at least 10pmol. Plate B was less expensive.

**Table 1: Binding of Eu-labelled test probe to compare streptavidin plates (counts/sec).**

| Probe concentration (log moles) | -13 | -12 | -11 | -10 | Bg (30min) |
|---|---|---|---|---|---|
| Plate A | 6.5E7 | 7.6E8 | 2.2E9 | 5.4E8 | 332 (514) |
| Plate B | 8.7E7 | 8.9E8 | 7.7E9 | 1.3E9 | 290 (362) |
| Plate C | 8.4E7 | 8.4E8 | 8.9E9 | 3.0E9 | 1275 (1244) |

A dilution series of test probe was also used to measure binding linearity at low levels as shown in Table 2. The same amount of at each dilution was also measured directly.

Linearity of binding was down to at least 10amol and only constrained at 10-100fmol where the high counting rate not being capable of resolved by the counter.

**Table 2: Binding of test probe to show binding linearity at low concentrations.**

| Moles of probe | -17 | -16 | -15 | -14 | -13 |
|---|---|---|---|---|---|
| Bound cps | 7.4E3 | 7.6E4 | 6.9E5 | 8.8E6 | 2.6E7 |
| Maximum | 2.4E4 | 2.5E5 | 2.2E6 | 1.8E7 | 2.8E7 |
| % bound | 32 | 33 | 35 | 52 | 104 (non-linear counting) |

### Example 2: Hybridisation

In methods of the invention, there is reliance on hybridisation of short oligonucleotide probes to resultant immobilised single stranded DNA. To make sure this aspect of the detection procedure was adequate, two probes were obtained with different random sequences, one labelled with Europium and the other Samarium as shown in the list of probes and oligonucleotides. In addition, two 5'biotinylated 79mer oligonucleotides were synthesised having the same random sequence, apart from 3'complementary sequences, one detectable by the Eu and the other by the Sm-labelled probe.

### Test Sm Oligo

SmGAACTCTCCTCG (SEQ ID NO: 2)

3′CTTGAGAGGAGCCCCGATGTTTTGGTGACGAAAGAATTACGCAAGGAGCGCTCAG
CAGGCTACGACTTCTCCATTCCCG-Bio (SEQ ID NO: 3)

### Test Eu Oligo

EuGGATATCACCCG (SEQ ID NO: 4)

3′CCTATAGTGGGCCCCGATGTTTTGGTGACGAAAGAATTACGCAAGGAGCGCTCAG
CAGGCTACGACTTCTCCATTCCCG-Bio (SEQ ID NO: 5)

These targets were immobilised at various concentrations on streptavidin-coated plates (A or B) and using the Eu-labelled probe at 4ng/200ul well and 10ng for Sm. The hybridisation buffer was again the commercial TRIS assay buffer from Wallac Oy, Finland, diluted 1:1 with deionised water and with the addition of 2M NaCl. Incubations at RT for 60 mins were followed by 6x washes in Wallac TRIS-based washing solution diluted 1:1 with deionised water. 200ul enhancement was added and the plate shaken for 30 min before counting.

**Table 3: Binding (cps) of 4ng Eu probe or 10ng Sm probe to immobilised 79-mers**

| 79mer concentration log mol | -12 | -13 | -14 | -15 |
|---|---|---|---|---|
| Eu probe plate A | 2.1E6 | 3.2E5 | 3.4E4 | 2.7E3 |
| Eu probe plate B | 2.6E6 | 4.7E5 | 1.5E4 | 1.4E3 |
| Sm probe plate A | 7.8E4 | 9.5E3 | 9.3E2 | ND |
| Sm probe plate B | 9.9E4 | 1.2E4 | 6.9E2 | ND |

The high cps and range available make this assay suitable for demonstrating the invention. The availability of these probes and oligonucleotides also allowed examination of other steps that would be used in the detection procedures for their optimisation. These steps will be familiar to those skilled in the art but are offered as reassurance that the assay used here can be regarded robust.

One aspect is the pH of the hybridisation buffer. A series of 12 TRIS buffers were made up to formula giving pH ranging from 8 to 9. These were measured and found in good agreement with that expected. Salt was added to 2M and bovine serum albumin to 0.2% in order to reduce non-specific binding. Binding of Eu-labelled probe to the 5' biotinylated 79mer oligonucleotides immobilised to streptavidin plates changed by only 20%, decreasing from pH8 to pH9. In some assays DNA denatured by the presence of 200mM NaOH was added to the buffer in aliquots that amounted to 5% of the final volume. The buffering action allowed the pH to remain in the range pH8-9 and hence had no significant effect on hybridisation.

Occasionally EDTA chelator may be needed to reduce Mg⁺⁺ ions in order to reduce the effect of any remaining Mg⁺⁺ on promoting DNA polymerase activity. It was found that for buffers containing 0.5M or 2M salt, that concentrations of 50mM or below of EDTA did not affect non-specific binding

Another variable is the stringency used both for hybridisation and for washing the plates free of unbound material. For these hybridisations, salt was added to the Wallac buffer, previously diluted 1:1 with water up to 1M or 2M. Very little difference was seen in non-specific hybridisation background and hybridisation binding was little different as shown below in Table 4.

**Table 4: Hybridisation at 1 and 2M salt buffer for 4ng of Eu or 10ng Sm-labelled probes per well to biotinylated 79mers immobilised to a streptavidin coated plate.**

| cps (n=3) ± SD | 1M salt hybridisation | 2M salt hybridisation |
|---|---|---|
| Eu probe | 594733±23381 | 536304±19645 |
| Sm probe | 18542±592 | 17288±2418 |

For the washing efficiency the Wallac wash solution (150mM salt), was tested by dilution at 150, 100, 75, 50, 25 and 12.5mM using Sm-labelled probe at 10ng/well and Eu at 4ng/well to hybridise to biotinylated 79mer targets at 1pmol/well, immobilised to streptavidin plates for 60 mins. Samples of wells as strips of 12 were washed 6 times with the different wash solutions. The Sm bound non-specifically to the Eu targets was approximately 2% and showed no effect with NaCl concentration. The higher Eu cps showed a small reduction in non-specific binding with higher salt 0.033% at 12.5mM compared with 0.056% at 150mM.

Binding for both probes rose with salt concentration in the wash but was not much increased above the 50mM dilution as % of total label added as shown in Table 5. These are expressed as % of total label placed in each well monitored by adding an aliquot directly to uncoated wells containing enhancement solution.

**Table 5: Hybridisation of 4ng Eu or 10ng Sm-labelled probe to 1pmol biotinylated 79mer oligonucleotides immobilised to wells of a streptavidin coated plate.**

| | | | | | | |
|---|---|---|---|---|---|---|
| mM salt wash | 12.5 | 25 | 50 | 75 | 100 | 150 |
| % binding of Eu-probe | 14.5 | 16.7 | 18.4 | 18.9 | 19.5 | 19.9 |
| % binding of Sm-probe | 13.6 | 15.0 | 15.6 | 16.0 | 16.9 | 16.6 |

These experiments show that the labelling technique to be used to demonstrate the invention has good efficiency with high numerical precision and has low background and non-specific binding. It can also be used for double labelling in the same microtitration well. There is little counting spillover between the two counting channels of the different labels. This small interaction allows ratios of counts from the different labels to be used enabling internal controls or standards to be employed.

The short oligonucleotide probes used in the detection process for this invention will have relatively low melting points making dissociation after hybridisation possible and resulting in loss of signal. In the examples of probes used here, the melting point, according to the formula by R B Wallace et al in Nucleic-Acid Research, should be approximately 38° in the presence of 0.9M salt. When hybridised at room temperature (RT), which can obviously vary, there will be an equilibrium between binding and spontaneous dissociation through temperature exacerbated by low melting points in the oligonucleotides. When washing plates with low-salt washing solutions, much material could be lost with a resultant loss of signal and increased sample variance.

The loss during hybridisation was investigated by binding probe to the biotinylated target used above, for 60 mins at RT in the presence of 1M salt. 10fmol was used for the Eu-labelled probe and 100fmol for the Sm-labelled probe. The plate was then washed once and the wells refilled with the same buffer without probe. A zero time control was washed 6 x and Wallac enhancement solution added for 30 min while other wells were removed and washed at different times thereafter. The results shown below in Table 6, revealed a decrease in the amount of bound probe that stabilised to an exponential loss of about 6 % per hour as measured by time-resolved fluorometry.

**Table 6: Loss of 10fmol Eu-labelled probe and 100fmol Sm-labelled probe hybridised to 79mer targets immobilised to streptavidin plates by 5' biotin.**

| Time (min) | Eu-probe (10fmol/well)cps | Sm-probe(100fmol/well) cps |
|---|---|---|
| 0 | 21313 | 11165 |
| 30 | 14899 | 10601 |
| 90 | 14777 | 9409 |
| 150 | 11567 | 7597 |
| 210 | 11763 | 7541 |

This loss of material can be reduced by the addition of a low-temperature DNA polymerase to the well during hybridisation, along with the usual other reagents for DNA synthesis. The probe will then be extended along the immobilised target. As the numbers of bases synthesised increases, the binding will become tighter and less likely to dissociate spontaneously or through the washing step. As a result the washing procedure can be made more stringent and hence the assay will be improved in efficiency and robustness. An example of improved signal by this method is shown in Table 7 where about 19% higher signal was observed.

**Table 7: Effect of Klenov DNA polymerase extension of Eu 10fmol/well)(and Sm-labelled (100fmol/well) probes hybridised (1M salt) for 90min to 79mer targets immobilised to streptavidin plates by 5' biotin.**

| cps | Klenov probe extension | Control no extension |
|---|---|---|
| Eu-labelled probe | 13296 | 11163 |
| SD (n=2) | 499 | 403 |
| Sm-labelled probe | 13714 | 11512 |
| SD (n=2) | 613 | 491 |

For a similar situation but where the DNA is immobilised by binding to a target already attached to the plate in what is known as a sandwich detection system. The method described here could be used in a similar treatment but where some types of DNA polymerase would advance along to the target, and through their 5' 3'-endonuclease properties, could break the binding of the immobilised target fragment and defeat the object of the exercise. In such a case polymerase without this property such as the Klenov fragment should be used, with the advantage that this material is not expensive. Alternatively the extension could be restricted by addition to the well of chain terminator such as dideoxycytidine. Whenever this base is incorporated the synthesis will stop i.e. after 4 bases on average, but of course depending on the sequence involved. If it is included as a minority compared with the correct precursor deoxycytidine then statistically synthesis will continue until chance favours incorporation of the chain terminator. For example if there is one dideoxycytidine molecule for every 9 deoxycytidines the average chain length will terminate after 10 cytidines are added on average so the chain will be 10 times longer i.e. 40 bases rather than 4. By knowing the sequence the calculation can be made with confidence and the average extension made suitable for the purpose of increasing binding in an economical fashion whatever the ambient temperature but without disruption of the immobilisation in a sandwich detection system.

### Example 3: overhang synthesis and detection

This experiment used 1pmol each of two overhang primers, with the overhangs suitable for the Eu and Sm-labelled probes, together with 2pmol of a biotinylated common primer. A target suitable for amplification by the overhanging primers and detectable by either Eu or Sm probes to the respective overhangs was synthesised. This was also included at 1pmol. The primers are shown in the list below and had inner specific primers with melting points 53-54° so an annealing step of 50° was used in the initial amplification reaction.

### Primers and target

Overhang primer for Sm probe - 5' GAACTCTCCTCGCCCGATGTTTTGGTGA 3'
(MP 74.9, 53.7 w/o overhang) (SEQ ID NO: 6)
Overhang primer for Eu probe - 5' GGATATCACCCGCCCGATGTTTTGGTGA 3'
(MP 76.4, 53.7 w/o overhang) (SEQ ID NO: 7)
Common primer - 3' AGTCGTCCGATGCTGAAGAGGTAAGGGC-Biotin 5'
(MP 73.4, 53.4 w/o overhang) (SEQ ID NO: 8)
(Overhangs are underlined)
Target- 5'CCCGATGTTTTGGTGACGAAAGAATTACGCAAGGAGCGCTCAGCAGGCTACGACT 3' (SEQ ID NO: 9)

The procedure was to amplify conventionally with Taq polymerase (Promega Multimix), cycling at 94° for 30sec, 50° for 30sec and 72° synthesis for 1 min. After two such cycles the synthesis was expected to be as shown below (for the Sm-specific probe only). After this a similar single cycle but with a 30 second 65° annealing step should synthesise the overhangs, one of which would be complementary to the probes.

### Synthesis 1

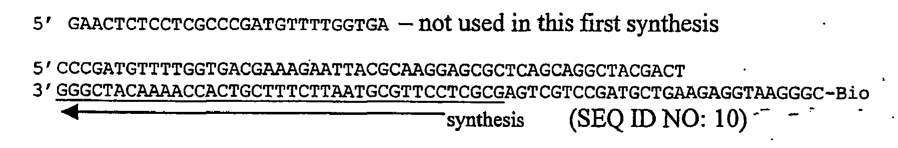
5' GAACTCTCCTCGCCCGATGTTTTGGTGA – not used in this first synthesis

5' CCCGATGTTTTGGTGACGAAAGAATTACGCAAGGAGCGCTCAGCAGGCTACGACT
3' GGGCTACAAAACCACTGCTTTCTTAATGCGTTCCTCGCGAGTCGTCCGATGCTGAAGAGGTAAGGGC-Bio
 synthesis (SEQ ID NO: 10)

### Synthesis 2, after melting

(SEQ ID NO: 11) synthesis
5' GAACTCTCCTCGCCCGATGTTTTGGTGACGAAAGAATTACGCAAGGAGCGCTCAGCAGGCTACGACTTCTCCATTCCCG
3' GGGCTACAAAACCACTGCTTTCTTAATGCGTTCCTCGCGAGTCGTCCGATGCTGAAGAGGTAAGGGC-
Bio

### Synthesis 3, after melting

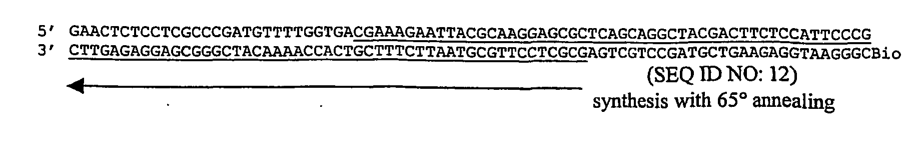
5' GAACTCTCCTCGCCCGATGTTTTGGTGACGAAAGAATTACGCAAGGAGCGCTCAGCAGGCTACGACTTCTCCATTCCCG
3' CTTGAGAGGAGCGGGCTACAAAACCACTGCTTTCTTAATGCGTTCCTCGCGAGTCGTCCGATGCTGAAGAGGTAAGGGCBio
(SEQ ID NO: 12)
synthesis with 65° annealing

### Detection, after alkali and wash to remove non-biotinylated strand

Eu-GAACTCTCCTCG
3' CTTGAGAGGAGCGGGCTACAAAACCACTGCTTTCTTAATGCGTTCCTCGCGAGTCGTCCGATGCTGAAGAGGTAAGGGC-
Bio

The experiment was performed with and without Taq polymerase and for both primers possessing the suitable overhangs for the two probes. The samples were incubated for 40 minutes in a streptavidin coated plate (Plate A). After incubation, the samples were washed 1x with wash solution, treated with 200mM NaOH and washed 1x. Hybridisation buffer and probe were then added. After 60 mins the plates were washed 6x and enhancement solution added for 30 min before counting by time-resolved fluorescence. The results are shown in Table 8. No activity was detected if Taq was absent showing that the non-specific probe did not bind significantly to the opposite synthesised targets. The presence of signals (shown in italic in Table 8) shows the new universal sequence was present, complementary to the probes and not present in the original target sequence.

**Table 8: Hybridisation of universal probe to newly synthesised universal overhang sequences as cps ± SD (n=2)**

| Targets | Sm + Taq | Sm - Taq | Eu + Taq | Eu - Taq |
|---|---|---|---|---|
| Mean Eu cps | 9018 | 7884 | *384063* | 8660 |
| ± SD | 1934 | 612 | 2040 | 516 |
| Mean Sm cps | *3047* | 325 | 730 | 296 |
| ± SD | 210 | 144 | 384 | 8 |

A second experiment was performed where the target was diluted and the Eu specific overhang primer used. The samples were subjected to two lower temperature-annealing cycles as before, followed by different numbers of higher annealing-temperature (65°) cycles to amplify the full-length product. The same detection procedure was followed using a plate B and the results shown in Table 9.

**Table 9: Hybridisation of universal probe to newly synthesised universal overhang sequences as cps for different target dilution vs. different numbers of higher temperature annealing amplification cycles**

| **cps of Eu** | Moles of target | | | | |
|---|---|---|---|---|---|
| No. cycles | 1E-11 | 1E-12 | 1E-13 | 1E-17 | 1E-21 |
| 1 | 1.6E6 | 1.6E4 | | | |
| 6 | | 1.9E6 | 2.1E4 | | |
| 11 | | 2.9E6 | 4.2E6 | 2.6E3 | |
| 16 | | | 3.2E6 | 3.4E3 | 1.5E4 |
| 21 | | | 4.3E6 | 3.1E4 | 2.3E4 |
| 26 | | | | 4.6E5 | 6.3E5 |
| 31 | | | | | 6.0E5 |

The results show clearly that extensive amplification has occurred since the cps rises after greater numbers of cycles as the target is progressively diluted and demonstrates that the basic invention is achieved.

## Claims

1. A method for detecting a target nucleic acid in a sample, which method comprises:
(a) providing a primer pair comprising a first primer having a target specific sequence complementary to the sequence of the first strand of said target nucleic acid and an overhang sequence unrelated to said target nucleic acid sequence and a second primer having a target specific sequence complementary to the sequence of the second strand of said target nucleic acid and an attachment means;
(b) contacting a nucleic acid sample with said first and second primers;
(c) carrying out a polymerase chain reaction (PCR) under conditions suitable for the formation of a double-stranded product comprising the sequence of said target nucleic acid and having a first strand comprising said first primer and a second strand comprising said second primer and a sequence complementary to said overhang sequence of said first primer;
(d) separating said first strand from said second strand using said attachment means; and then
(e) detecting said second strand by binding a probe to the sequence in said second strand which sequence is complementary to the overhang sequence in said first primer or detecting said first strand by binding said overhang sequence in said first strand to an immobilised anchor sequence and detecting the immobilised first strand
thereby detecting whether said target nucleic acid present in said sample.

2. A method according to claim 1, wherein step (d) comprises attaching said second strand to a surface via said attachment means.

3. A method according to claim 2, wherein step (d) further comprises denaturing said product and washing to remove removing any nucleic acid molecules not attached to said surface.

4. A method according to claim 2 or 3, wherein said second strand is detected in step (e).

5. A method according to claim 4, wherein said second strand is detected using a probe comprising a sequence identical to the sequence of the overhang in said first primer.

6. A method according to any one of the preceding claims, wherein said probe comprises a detectable label, which is optionally a chelate containing a rare earth element such as Europium, Samarium or Terbium.

7. A method according to claim 5 or 6, wherein said detecting comprises binding said probe to said second strand of product and monitoring said binding optionally by radioactive, fluorescent, chemiluminescent, colourimetric or time-resolved fluorescent means.

8. A method according to any one of the preceding claims, wherein said second primer comprises an overhang sequence.

9. A method according to any one of the preceding claims, wherein said attachment means on said second primer comprises a biotin moiety and step (d) comprises binding said second strand via said biotin moiety to a streptavidin-coated surface.

10. A method according to any one of claims 1 to 8, wherein said attachment means on said second primer comprises an overhang sequence complementary to an anchor sequence and step (d) comprises either binding said second strand via said overhang sequence to a surface on which an oligonucleotide comprising said anchor sequence is immobilised or binding said second strand via said overhang sequence to an oligonucleotide comprising said anchor sequence and immobilising said oligonucleotide to a surface.

11. A method according to any one of the preceding claims, wherein said first primer further comprises an attachment means.

12. A method according to claim 11, wherein said first primer and said second primer comprise the same attachment means.

13. A method according to claim 11, wherein the first primer comprises a different attachment means to said attachment means on said second strand.

14. A method according to claim 13, wherein said first primer and said first strand are removed by binding to magnetic beads and said first primer and said first strand are removed using magnetic means.

15. A method according to any one of the preceding claims, wherein a set of primers comprising two or more said primer pairs are provided for the detection of two or more target nucleic acids.

16. A method according to claim 15, wherein:
(i) each first primer in said set comprises a different overhang sequence and a different target specific sequence and each second primer in said set comprises a different target specific sequence but the same attachment means; and wherein
(ii) in step (e) each of the products produced using each of the primer pairs are detected using a different probe, each different probe having a different sequence and being labelled with a different label.

17. A method according to claim 15 or 16 for use in determining the relative abundance of two or more target nucleic acids.

18. A method according to any one of claims 15 to 17 wherein in step (c) each PCR using a different primer pair is carried out in the same reaction vessel.

19. A method according to claim 15, wherein:
(i) each first primer in said set has the same overhang sequence but a different target specific sequence and each second primer in said set comprises a different target specific sequence and a different attachment means.

20. A method according to claim 19, wherein:
(i) in step (d) each said second strand produced using each of the primer pairs is attached to a different surface
(ii) in step (e) each said second strand produced using each of the primer pairs is detected using the same probe; and the method further comprises:
(f) distinguishing the nucleic acids present in said sample on the basis of the surface to which detectable products are attached.

21. A method according to claim 20, wherein, said different surfaces are separate wells in a multiwell plate or separate spots on an array.

22. A method according to claim 20 or 21, wherein said attachment means are overhang sequences and said different surfaces have different anchor oligonucleotides immobilised thereon.

23. A method according to any one of the preceding claims for use in determining the presence of a single nucleotide polymorphism (SNP) in a sample from an individual.

24. A method according to claim 23, wherein two primer pairs are used to amplify two variants of a SNP, and the proportions of product produced using each of the primer pairs is monitored to determine whether said individual is homozygous or heterozygous for said SNP.

25. A method according to any one of claims 1 to 3 and 8 to 24, wherein the first strand of the PCR product is detected following removal of the second strand and wherein:
(i) in step (c) a label is incorporated into the PCR products;
(ii) in step (e) said label is detected.

26. A method according to claim 25, wherein:
(i) in step (d) said second strands and said second primers are removed by attaching said strands to beads via said attachment means;
(ii) in step (d) said first strands are attached to a surface via binding of said overhang region to an anchor sequence immobilised on said surface; and
(iii) in step (e) said first strands are detected.

27. A kit for detecting a target nucleic acid molecule, which kit comprises:
(a) an oligonucleotide probe comprising the same sequence as an overhang sequence of a first primer having a target specific sequence complementary to the sequence of the first strand of said target nucleic acid and an overhang sequence unrelated to said target nucleic acid sequence;
(b) an immobilisation surface coated with streptavidin;
(c) a primer pair suitable for use in detecting a target nucleic acid, which primer pair comprises:
(i) a first primer having a target specific sequence complementary to the sequence of the first strand of said target nucleic acid and an overhang sequence unrelated to said target nucleic acid sequence; and
(ii) a second primer having a target specific sequence complementary to the sequence of the second strand of said target nucleic acid and an attachment means, wherein said attachment means is biotin.

28. A method according to any one of claims 1 to 26, which further comprises selecting the overhang sequence of the primer by:
(a) generating all possible sequences for a particular suitable length of overhang;
(b) rejecting every sequence found within the sequence of interest;
(c) optionally rejecting sequences having secondary structures;
(d) selecting the remaining sequences for use in the generation of primers.

29. A method according to claim 28, wherein the sequence of interest is the sequence of a whole genome.

## Patentansprüche

1. Verfahren zum Nachweisen einer Zielnukleinsäure in einer Probe, welches Verfahren umfasst:
(a) Bereitstellen eines Primerpaars, umfassend einen ersten Primer mit einer Ziel-spezifischen Sequenz, die zu der Sequenz des ersten Strangs der Zielnukleinsäure komplementär ist, und einer Überhangsequenz, die mit der Zielnukleinsäuresequenz nicht verwandt ist, und einen zweiten Primer mit einer Ziel-spezifischen Sequenz, die zu der Sequenz des zweiten Strangs der Zielnukleinsäure komplementär ist, und einem Anlagerungselement;
(b) Kontaktieren einer Nukleinsäure-Probe mit den ersten und zweiten Primern;
(c) Durchführen einer Polymerase-Kettenreaktion (PCR) unter Bedingungen, die für die Bildung eines doppelsträngigen Produkts, umfassend die Sequenz der Zielnukleinsäure, geeignet sind, und welches einen ersten Strang, umfassend den ersten Primer, und einen zweiten Strang, umfassend den zweiten Primer und eine Sequenz, die zu der Überhangsequenz des ersten Primers komplementär ist, aufweist;
(d) Trennen des ersten Strangs von dem zweiten Strang unter Verwendung des Anlagerungselements; und dann
(e) Nachweisen des zweiten Strangs durch Binden einer Sonde an die Sequenz in dem zweiten Strang, welche Sequenz zu der Überhangsequenz in dem ersten Primer komplementär ist, oder Nachweisen des ersten Strangs durch Binden der Überhangsequenz in dem ersten Strang an eine immobilisierte Ankersequenz und Nachweisen des immobilisierten ersten Strangs, dabei Nachweisen, ob die Zielnukleinsäure in der Probe vorhanden ist.

2. Verfahren nach Anspruch 1, wobei Schritt (d) das Anlagern des zweiten Strangs an eine Oberfläche über das Anlagerungselement umfasst:

3. Verfahren nach Anspruch 2, wobei Schritt (d) außerdem das Denaturieren des Produkts und Waschen zur Entfernung jeglicher nicht an die Oberfläche angelagerter Nukleinsäure-Moleküle umfasst.

4. Verfahren nach Anspruch 2 oder 3, wobei der zweite Strang in Schritt (e) nachgewiesen wird.

5. Verfahren nach Anspruch 4, wobei der zweite Strang unter Verwendung einer Sonde nachgewiesen wird, umfassend eine Sequenz, die zu der Sequenz des Überhangs in dem ersten Primer identisch ist.

6. Verfahren nach einem der vorangehenden Ansprüche, wobei die Sonde eine nachweisbare Markierung umfasst, die wahlweise ein Chelat ist, das ein Seltenerdelement, wie etwa Europium, Samarium oder Terbium, enthält.

7. Verfahren nach Anspruch 5 oder 6, wobei das Nachweisen das Binden der Sonde an den zweiten Strang des Produkts und das Überwachen der Bindung, wahlweise mittels radioaktiver, fluoreszierender, chemilumineszierender, kolorimetrischer oder zeitlich aufgelöster fluoreszierender Elemente, umfasst.

8. Verfahren nach einem der vorangehenden Ansprüche, wobei der zweite Primer eine Überhangsequenz umfasst.

9. Verfahren nach einem der vorangehenden Ansprüche, wobei das Anlagerungselement auf dem zweiten Primer eine Biotin-Komponente umfasst und Schritt (d) das Binden des zweiten Strangs über die Biotin-Komponente an eine Streptavidin-beschichtete Oberfläche umfasst.

10. Verfahren nach einem der Ansprüche 1 bis 8, wobei das Anlagerungselement auf dem zweiten Primer eine Überhangsequenz umfasst, die zu einer Ankersequenz komplementär ist, und Schritt (d) entweder das Binden des zweiten Strangs über die Überhangsequenz an eine Oberfläche, an der ein Oligonukleotid, das die Ankersequenz umfasst, immobilisiert ist, oder das Binden des zweiten Strangs über die Überhangsequenz an ein Oligonukleotid, das die Ankersequenz umfasst, und das Immobilisieren des Oligonukleotids an einer Oberfläche umfasst.

11. Verfahren nach einem der vorangehenden Ansprüche, wobei der erste Primer außerdem ein Anlagerungselement umfasst.

12. Verfahren nach Anspruch 11, wobei der erste Primer und der zweite Primer das selbe Anlagerungselement umfassen.

13. Verfahren nach Anspruch 11, wobei der erste Primer ein verschiedenes Anlagerungselement zu dem Anlagerungselement auf dem zweiten Strang umfasst.

14. Verfahren nach Anspruch 13, wobei der erste Primer und der erste Strang durch Binden an Magnetkügelchen entfernt werden, und der erste Primer und der erste Strang unter Verwendung eines magnetischen Elements entfernt werden.

15. Verfahren nach einem der vorangehenden Ansprüche, wobei ein Satz von Primern, umfassend zwei oder mehr der Primerpaare, für den Nachweis von zwei oder mehr Zielnukleinsäuren bereitgestellt wird.

16. Verfahren nach Anspruch 15, wobei:
(i) jeder erste Primer in dem Satz eine unterschiedliche Überhangsequenz und eine unterschiedliche Ziel-spezifische Sequenz umfasst, und jeder zweite Primer in dem Satz eine unterschiedliche Ziel-spezifische Sequenz, doch das selbe Anlagerungselement umfasst; und worin
(ii) in Schritt (e) jedes der Produkte, das unter Verwendung jedes der Primerpaare erzeugt wurde, unter Verwendung einer unterschiedlichen Sonde nachgewiesen wird, wobei jede unterschiedliche Sonde eine unterschiedliche Sequenz aufweist und mit einer unterschiedlichen Markierung markiert ist.

17. Verfahren nach Anspruch 15 oder 16, zur Verwendung in der Bestimmung der relativen Häufigkeit von zwei oder mehr Zielnukleinsäuren.

18. Verfahren nach einem der Ansprüche 15 bis 17, wobei in Schritt (c) jede PCR unter Verwendung eines unterschiedlichen Primerpaars in dem gleichen Reaktionsgefäß vorgenommen wird.

19. Verfahren nach Anspruch 15, wobei:
(i) jeder erste Primer in dem Satz die selbe Überhangsequenz, doch eine unterschiedliche Ziel-spezifische Sequenz aufweist und jeder zweite Primer in dem Satz eine unterschiedliche Ziel-spezifische Sequenz und ein unterschiedliches Anlagerungselement aufweist.

20. Verfahren nach Anspruch 19, wobei:
(i) in Schritt (d) jeder besagte zweite Strang, der unter Verwendung jedes der Primerpaare erzeugt wird, an eine unterschiedliche Oberfläche angelagert wird
(ii) in Schritt (e) jeder besagte zweite Strang, der unter Verwendung jedes der Primerpaare erzeugt wird, unter Verwendung der selben Sonde nachgewiesen wird; und welches Verfahren weiterhin umfasst:
(f) Unterscheiden der in der Probe vorhandenen Nukleinsäuren ausgehend von der Oberfläche, an die nachweisbare Produkte angelagert sind.

21. Verfahren nach Anspruch 20, wobei die unterschiedlichen Oberflächen separate Wells in einer Multiwellplatte oder separate Flecken auf einem Array sind.

22. Verfahren nach Anspruch 20 oder 21, wobei das Anlagerungselement Überhangsequenzen sind und die unterschiedlichen Oberflächen unterschiedliche, daran immobilisierte Anker-Oligonukleotide aufweisen.

23. Verfahren nach einem der vorangehenden Ansprüche, zur Verwendung in der Bestimmung des Vorhandenseins eines Einzel-Nukleotid-Polymorphismus (SNP) in einer Probe von einem Individuum.

24. Verfahren nach Anspruch 23, wobei zwei Primerpaare zur Amplifikation zweier Varianten eines SNP verwendet werden, und die Anteile an Produkt, die unter Verwendung jedes der Primerpaare erzeugt wurden, überwacht werden, um zu bestimmen, ob das Individuum homozygot oder heterozygot für den SNP ist.

25. Verfahren nach einem der Ansprüche 1 bis 3 und 8 bis 24, wobei der erste Strang des PCR-Produkts nach Entfernung des zweiten Strangs nachgewiesen wird, und wobei:
(i) in Schritt (c) eine Markierung in die PCR-Produkte aufgenommen wird;
(ii) in Schritt (e) die Markierung nachgewiesen wird.

26. Verfahren nach Anspruch 25, wobei:
(i) in Schritt (d) die zweiten Stränge und die zweiten Primer durch Anlagern der Stränge an Kügelchen über das Anlagerungselement entfernt werden;
(ii) in Schritt (d) die ersten Stränge an eine Oberfläche über die Bindung der Überhangregion an eine Ankersequenz, die an der Oberfläche immobilisiert ist, angelagert werden; und
(iii) in Schritt (e) die ersten Stränge nachgewiesen werden.

27. Kit zum Nachweisen eines Zielnukleinsäure-Moleküls, welcher Kit umfasst:
(a) eine Oligonukleotid-Sonde, umfassend die selbe Sequenz wie eine Überhangsequenz eines ersten Primers, der eine Ziel-spezifische Sequenz, die zu der Sequenz des ersten Strangs der Zielnukleinsäure komplementär ist, und eine Überhangsequenz, die mit der Zielnukleinsäuresequenz nicht verwandt ist, aufweist;
(b) eine Immobilisierungsoberfläche, die mit Streptavidin beschichtet ist;
(c) ein Primerpaar, das zur Verwendung beim Nachweisen einer Zielnukleinsäure geeignet ist, welches Primerpaar umfasst:
(i) einen ersten Primer mit einer Ziel-spezifischen Sequenz, die zu der Sequenz des ersten Strangs der Ziel-Nukleinsäure komplementär ist, und einer Überhangsequenz, die zu der Ziel-Nukleinsäuresequenz nicht verwandt ist; und
(ii) einen zweiten Primer mit einer Ziel-spezifischen Sequenz, die zu der Sequenz des zweiten Strangs der Zielnukleinsäure komplementär ist, und einem Anlagerungselement, wobei das Anlagerungselement Biotin ist.

28. Verfahren nach einem der Ansprüche 1 bis 26, welches außerdem das Selektieren der Überhangsequenz des Primers umfasst, indem:
(a) alle möglichen Sequenzen für eine bestimmte, geeignete Länge des Überhangs erzeugt werden;
(b) jede Sequenz, die innerhalb der Sequenz von Interesse gefunden wird, ausgeschlossen wird;
(c) wahlweise Sequenzen mit Sekundärstrukturen ausgeschlossen werden;
(d) die verbliebenen Sequenzen zur Verwendung in der Erzeugung von Primern selektiert werden.

29. Verfahren nach Anspruch 28, wobei die Sequenz von Interesse die Sequenz eines Gesamtgenoms ist.

## Revendications

1. Procédé de détection d'un acide nucléique cible dans un échantillon, lequel procédé comporte :
a) le fait de prendre une paire d'amorces constituée d'une première amorce qui comporte une séquence spécifique pour cible, complémentaire de la séquence du premier brin dudit acide nucléique cible, et une séquence protubérante sans rapport avec la séquence dudit acide nucléique cible, et d'une deuxième amorce qui comporte une séquence spécifique pour cible, complémentaire de la séquence du deuxième brin dudit acide nucléique cible, et un moyen d'attache ;
b) le fait de mettre un échantillon d'acide nucléique en contact avec lesdites première et deuxième amorces ;
c) le fait d'effectuer une amplification en chaîne par polymérase (PCR) dans des conditions appropriées pour la formation d'un produit double-brin qui comprend la séquence dudit acide nucléique et dont le premier brin comporte ladite première amorce et le deuxième brin comporte ladite deuxième amorce et une séquence complémentaire de ladite séquence protubérante de ladite première amorce ;
d) le fait de séparer ledit premier brin dudit deuxième brin, à l'aide dudit moyen d'attache ;
e) et puis le fait de détecter ledit deuxième brin, par liaison d'une sonde à la séquence, présente dans ledit deuxième brin, qui est complémentaire de ladite séquence protubérante de ladite première amorce, ou le fait de détecter ledit premier brin, par liaison de ladite séquence protubérante, présente dans ledit premier brin, à une séquence d'ancrage immobilisée et détection dudit premier brin immobilisé ;
ce qui permet de déterminer si ledit acide nucléique cible se trouve présent dans ledit échantillon.

2. Procédé conforme à la revendication 1, dans lequel l'étape (d) comporte le fait d'attacher ledit deuxième brin à une surface, à l'aide dudit moyen d'attache.

3. Procédé conforme à la revendication 2, dans lequel l'étape (d) comporte en outre le fait de dénaturer ledit produit et un lavage qui permet d'éliminer toute molécule d'acide nucléique qui n'est pas attachée à ladite surface.

4. Procédé conforme à la revendication 2 ou 3, dans lequel c'est le deuxième brin qu'on détecte dans l'étape (e).

5. Procédé conforme à la revendication 4, dans lequel on détecte ledit deuxième brin à l'aide d'une sonde comprenant une séquence identique à ladite séquence protubérante présente dans la première amorce.

6. Procédé conforme à l'une des revendications précédentes, dans lequel ladite sonde comprend un marqueur détectable, qui est éventuellement un chélate contenant un élément du groupe des terres rares, tel que l'europium, le samarium ou le terbium.

7. Procédé conforme à la revendication 5 ou 6, dans lequel ladite opération de détection comporte le fait de lier ladite sonde audit deuxième brin du produit et le fait de surveiller cette liaison par des moyens faisant appel, au choix, à la radioactivité, à la fluorescence, à la chemioluminescence, à la colorimétrie ou à la fluorescence résolue en temps.

8. Procédé conforme à l'une des revendications précédentes, dans lequel ladite deuxième amorce comporte une séquence protubérante.

9. Procédé conforme à l'une des revendications précédentes, dans lequel ledit moyen d'attache présent dans ladite deuxième amorce com-prend un motif biotine et l'étape (d) comporte le fait de lier ledit deuxième brin, par l'intermédiaire de ce motif biotine, à une surface portant de la streptavidine.

10. Procédé conforme à l'une des revendications 1 à 8, dans lequel ledit moyen d'attache présent dans ladite deuxième amorce comprend une séquence protubérante complémentaire d'une séquence d'ancrage et l'étape (d) comporte soit le fait de lier ledit deuxième brin, par l'intermédiaire de cette séquence protubérante, à une surface sur laquelle est immobilisé un oligonucléotide comportant ladite séquence d'ancrage, soit le fait de lier ledit deuxième brin, par l'intermédiaire de cette séquence protubérante, à un oligonucléotide comportant ladite séquence d'ancrage et le fait d'immobiliser cet oligonucléotide sur une surface.

11. Procédé conforme à l'une des revendications précédentes, dans lequel ladite première amorce comporte en outre un moyen d'attache.

12. Procédé conforme à la revendication 11, dans lequel ladite première amorce et ladite deuxième amorce comportent le même moyen d'attache.

13. Procédé conforme à la revendication 11, dans lequel la première amorce comporte un moyen d'attache différent du moyen d'attache de ladite deuxième amorce.

14. Procédé conforme à la revendication 13, dans lequel ladite première amorce et ledit premier brin sont attachés à des billes magnétiques et séparés à l'aide de moyens magnétiques.

15. Procédé conforme à l'une des revendications précédentes, dans lequel on prend un jeu d'amorces comprenant deux paires d'amorces dudit type ou plus, afin de détecter deux acides nucléiques cibles ou plus.

16. Procédé conforme à la revendication 15, dans lequel :
i) chaque première amorce dudit jeu comporte une séquence protubérante différente et une séquence spécifique pour cible différente, et chaque deuxième amorce dudit jeu comporte une séquence spécifique pour cible différente, mais le même moyen d'attache ;
ii) et dans l'étape (e), chacun des produits obtenus à l'aide de toutes les paires d'amorces est détecté au moyen d'une sonde différente, chacune de ces différentes sondes comprenant une séquence différente et se trouvant marquée avec une marqueur différent.

17. Procédé conforme à la revendication 15 ou 16, servant à déterminer l'abondance relative de deux acides nucléiques cibles ou plus.

18. Procédé conforme à l'une des revendications 15 à 17, dans lequel, dans l'étape (c), on effectue dans le même récipient réactionnel toutes les opérations de PCR réalisées avec des paires d'amorces différentes.

19. Procédé conforme à la revendication 15, dans lequel :
i) chaque première amorce dudit jeu comporte la même séquence protubérante, mais une séquence spécifique pour cible différente, et chaque deuxième amorce dudit jeu comporte une séquence spécifique pour cible différente et un moyen d'attache différent.

20. Procédé conforme à la revendication 19, dans lequel :
i) dans l'étape (d), chacun desdits deuxièmes brins produits à l'aide de chacune des paires d'amorces est attaché à une surface différente ;
ii) et dans l'étape (e), chacun desdits deuxièmes brins produits à l'aide de chacune des paires d'amorces est détecté à l'aide de la même sonde ;
et lequel procédé comporte en outre
f) le fait de distinguer les uns des autres les acides nucléiques présents dans ledit échantilllon en se basant sur les surfaces auxquelles les produits détectables sont attachés.

21. Procédé conforme à la revendication 20, dans lequel lesdites surfaces différentes sont les puits distincts d'une plaque multipuits ou les points distincts d'un réseau.

22. Procédé conforme à la revendication 20 ou 21, dans lequel lesdits moyens d'attache sont des séquences protubérantes et lesdites sur-faces différentes portent différents oligonucléotides d'ancrage immobilisés.

23. Procédé conforme à l'une des revendications précédentes, servant à déterminer la présence d'un polymorphisme à un seul nucléotide (SNP) dans un échantillon en provenance d'un individu.

24. Procédé conforme à la revendication 23, dans lequel on utilise deux paires d'amorces pour amplifier deux variants d'un SNP, et l'on surveille les proportions des produits fournis par chacune des paires d'amorces afin de déterminer si ledit individu est homozygote ou hétérozygote pour ledit SNP.

25. Procédé conforme à l'une des revendications 1 à 3 et 8 à 24, dans lequel le premier brin du produit de PCR est détecté après élimination du deuxième brin, et dans lequel :
i) dans l'étape (c), on incorpore un marqueur aux produits de PCR ;
ii) dans l'étape (e), on détecte ce marqueur.

26. Procédé conforme à la revendication 25, dans lequel :
i) dans l'étape (d), on élimine lesdits deuxièmes brins et lesdites deuxièmes amorces en attachant lesdits brins à des billes, par l'intermédiaire desdits moyens d'attache ;
ii) dans l'étape (d), on attache lesdits premiers brins à une surface, par liaison de ladite séquence protubérante à une séquence d'ancrage immobilisée sur ladite surface ;
iii) et dans l'étape (e), on détecte lesdits premiers brins.

27. Trousse servant à détecter un acide nucléique cible, laquelle trousse comprend :
a) une sonde oligonucléotidique comportant une séquence identique à une séquence protubérante d'une première amorce qui comporte une séquence spécifique pour cible, complémentaire de la séquence du premier brin dudit acide nucléique cible, et une séquence protubérante sans rapport avec la séquence dudit acide nucléique cible ;
b) une surface d'immobilisation, qui porte de la streptavidine ;
c) une paire d'amorces appropriées pour servir à la détection d'un acide nucléique cible, laquelle paire d'amorces comporte :
i) une première amorce comportant une séquence spécifique pour cible, complémentaire de la séquence du premier brin dudit acide nucléique cible, et une séquence protubérante sans rapport avec la séquence dudit acide nucléique cible,
ii) et une deuxième amorce comportant une séquence spécifique pour cible, complémentaire de la séquence du deuxième brin dudit acide nucléique cible, et un moyen d'attache, lequel moyen d'attache est de la biotine.

28. Procédé conforme à l'une des revendications 1 à 26, qui comporte en outre le fait de sélectionner la séquence protubérante de l'amorce :
a) en générant toutes les séquences possibles pour une certaine longueur convenable de la séquence protubérante ;
b) en rejetant toute séquence qui se trouve au sein de la séquence d'intérêt ;
c) en rejetant, le cas échéant, les séquences présentant des structures secondaires ;
d) et en choisissant les séquences restantes pour s'en servir pour générer des amorces.

29. Procédé conforme à la revendication 28, dans lequel la séquence d'intérêt est la séquence d'un génome entier.


### SEQUENCE LISTING

<110> BioQuant Limited
   Colin Gerard Potter
<120> UNIVERSAL PROBE SYSTEM
<130> N83305 GCW
<140>
   <141> 2001-08-07
<150> GB 0019179.1
   <151> 2000-08-07
<160> 12
<210> 1
   <211> 13
   <212> -DNA
   <213> Artificial Sequence
<220
   <221> misc_feature
   <223> Description of Artificial Sequence: probe
<400> 1
   taataagaca tcc 13
<210> 2
   <211> 12
   <212> DNA
   <213> Artificial Sequence
<220>
   <221> misc_feature
   <223> Description of Artificial Sequence: Probe
<400> 2
   gaactctcct cg 12
<210> 3
   <211> 79
   <212> DNA
   <213> Artificial Sequence
<220>
   <221> misc_feature
   <223> Description of Artificial Sequence: test target sequence
<400> 3
   gcccttacct cttacagcatc ggacgactcg cgaggaacgc attaagaaag cagtggtttt 60
   gtagccccga ggagagttc 79
<210> 4
   <211> 12
   <212> DNA
   <213> Artificial Sequence
<220>
   <221> misc_feature
   <223> Description of Artificial Sequence: Probe
<400> 4
   ggatatcacc cg 12
<210> 5
   <211> 79
   <212> DNA
   <213> Artificial Sequence
<220>
   <221> misc_feature
   <223> Description of Artificial Sequence: test target sequence
<400> 5
   gcccttacct cttcagcatc ggacgactcg cgaggaacgc attaagaaag cagtggtttt 60
   gtagccccgg gtgatatcc 79
<210> 6
   <211> 28
   <212> DNA
   <213> Artificial Sequence
<220>
   <221> misc_feature
   <223> Description of Artificial Sequence: primer
<400> 6
   gaactctcct cgcccgatgt tttggtga 28
<210> 7
   <211> 28
   <212> DNA
   <213> Artificial Sequence
<220>
   <221> misc_feature
   <223> Description of Artificial Sequence: Primer
<400> 7
   ggatatcacc cgcccgatgt tttggtga 28
<210> 8
   <211> 28
   <212> DNA
   <213> Artificial Sequence
<220>
   <221> misc_feature
   <223> Description of Artificial Sequence: Primer
<400> 8
   cgggaatgga gaagtcgtag cctgctga 28
<210> 9
   <211> 55
   <212> DNA
   <213> Artificial Sequence
<220>
   <221> misc_feature
   <223> Description of Artificial Sequence: Target sequence
<400> 9
   eccgatgttt tggtgacgaa agaattacgc aaggagcgct cagcaggcta cgact 55
<210> 10
   <211> 67
   <212> DNA
   <213> Artificial Sequence
<220>
   <221> misc_feature
   <223> Description of Artificial Sequence: Intermediate PCR product
<400> 10
   cgggaatgga gaagtcgtag cctgctgagc gctccttgcg taattctttc gtcaccaaaa 60
   catcggg 67
<210> 11
   <211> 79
   <212> DNA
   <213> Artificial Sequence
<220>
   <221> misc_feature
   <223> Description of Artificial Sequence: PCR product
<400> 11
   gaactctcct cgcccgatgt tttggtgacg aaagaattac gcaaggagcg ctcagcaggc 60
   tacgacttct ccattcccg 79
<210> 12
   <211> 79
   <212> DNA
   <213> Artificial Sequence
<220>
   <221> misc_feature
   <223> Description of Artificial Sequence: PCR product
<400> 12
   cgggaatgga gaagtcgtag cctgctgagc gctccttgcg taattctttc gtcaccaaaa 60
   catcgggcga ggagagttc 79